# EUROPEAN PATENT SPECIFICATION

(11) **EP 1 345 556 B1**
(45) Date of publication and mention of the grant of the patent: **10.09.2008**
(21) Application number: 01997248.8
(22) Date of filing: 20.11.2001
(51) Int. Cl.: A61F 2/06, A61B 17/12

(54) **OCCLUSION DEVICE**
OKKLUSIONSVORRICHTUNG
DISPOSITIF D'OCCLUSION

(30) Priority: 25.11.2000 US 722206
(43) Date of publication of application: 24.09.2003
(73) Proprietor: Board of Regents, The University of Texas System, Austin Texas 78701 (US)
(72) Inventor: KONYA, András, Houston, TX 77096 (US); WRIGHT, Kenneth, C., Houston, TX 77006 (US)
(74) Representative: Dehmel, Albrecht
(86) International application number: PCT/US2001/043298
(87) International publication number: WO 2002/041753

(56) References cited:
- US-A- 5 382 259
- US-A- 5 383 887
- US-A- 5 830 230
- US-A- 5 941 896
- US-A- 5 954 745

## Description

### BACKGROUND OF THE INVENTION

### 1. Field of the Invention

The present invention relates generally to the field of passageway occlusion within living beings. More particularly, it concerns repositionable and, in some cases, retrievable devices useful for achieving vascular occlusion.

### 2. Description of Related Art

The most widely-used permanent vascular occluding (i.e., clotting) device has long been the Gianturco stainless steel coil (Cook Inc., Bloomington, IN), which, since its introduction in the mid-1970s, has undergone several modifications regarding its thrombogenicity (i.e., its effectiveness in causing blood clotting) and delivery system (1-3). These devices, also known as macrocoils (0.035" - 0.038"), (0,0889 cm - 0,0965 cm) have been widely used for vessel occlusions throughout the body. For neurologic embolizations, several types of microcoils (0.010" - 0.018'") (0,0254 cm - 0,0457 cm) (Cook Inc, Bloomington, IN; Medi-Tech, a division of Boston Scientific Co., Natick, MA) have been developed. One disadvantage of these embolic coils is that they tend to migrate if they are used in veins or in non-tapered vascular structures. Additionally, high-flow vascular lesions including arterio-venous malformations (AVMs) and fistulas increase the possibility of coil dislodgment even further (4-6).

There have been several attempts to offset this disadvantage of coil migration after placement, otherwise known as coil emboli. The "coil in coil technique" uses a smaller coil released into the lumen of a larger coil to decrease the length of the occluded vessel segment and also prevent coil migration (7). Coils have also been equipped with a single barb to anchor the coil when placed on the venous side (8). The separately-deployed Amplatz spider was designed to prevent coil dislodgment both in the arterial (9) and the venous side (10). It could be delivered downstream of the delivered coils, and was designed to capture them should they migrate. Some authors have recently proposed the use of a nitinol snare to improve transcatheter delivery of macrocoils in occlusion of patent ductus arteriosus (PDA) (11,12).

Additionally, detachable coils have been designed to prevent migration by allowing optimal positioning of the coil before its release. For neurologic interventions, several microcoil systems equipped with a controlled delivery mechanism have been developed. The most widely used system is the Guglielmi electrically detachable microcoil (Medi-Tech) (13-15). Mechanically detachable microcoils (e.g., interlocking detachable coil systems) are also available (Medi-Tech) (16, 17). Further, the use of controlled-release macrocoils (Cook detachable coil; William Cook Europe, Bjeverskov, Denmark) with a spiral releasing mechanism is increasing (18-20). The inventors of the present invention have published their first experimental results with a new anchoring coil system (37). Even more recently, a similar anchoring coil system has been presented in Japan (38).

In US patent 5,941,896 a filter and method for trapping emboli during endovascular procedures are provided. In one embodiment of this teaching, the filter is formed from a bent, flexible guidewire shaped to define a frame and a porous filtering material mounted to portions thereof. In a collapsed state, the filter can readily pass through the lumen of a catheter and into the bloodstream of a patient. Upon completion of an endovascular procedure, the filter is collapsed and retracted into the catheter. In alternative embodiments of US patent 5,941,896, porous filtering material is mounted to external portions of a catheter, and a control guidewire is provided to selectively expand the filter between open and closed states.

US 5,830,230 discloses a method of intracranial placement of an embolization coil provided with an anchor to prevent migration of the coil after placement. The occlunion device disclosed in this document comprises an anchor and an occludes as defined as defined in claim 1.

Although early experiences with the above-mentioned systems are favorable (37,38), problems still remain. Foremost among them occurs when the anchors for the systems are misplaced: the misplaced anchors carmot be retrieved or repositioned. As a result of this shortcoming, the functionality and reliability of these systems is compromised in many clinical situations, narrowing the acceptable scope of their use.

The problems pointed out with the foregoing occlusion devices are not intended to be exhaustive but rather are among many that tend to impair the effectiveness of previously known occlusion devices. Other noteworthy problems may also exist; however, those presented above should be sufficient to demonstrate that previous techniques appearing in the art have not been altogether satisfactory, particularly in providing an occlusion device that offers reliable, correctable functionality.

### SUMMARY OF THE INVENTION

In an attempt to overcome the foregoing disadvantages, the inventors created the present occlusion devices and devised the following deployment system and methods for their deployment. The design of these occlusion devices simultaneously allow for their repositionability and retrievability while making it easy for the operator/surgeon to create the most appropriate arrangement of the occluder and anchor together.

In one respect, the present invention is an occlusion device as defined in claim 1. The occlusion device includes an anchor having a variable shape. An occluder is attached to the anchor at a first location, and a filament is attached to the anchor at a second location. Manipulation of the filament alters the variable shape of the anchor.

In other respects, the anchor may include a wire. The wire may be stainless steel. The wire may be a nickel titanium alloy. The anchor may include a wire having multiple acute bends. The anchor may include a wire having two ends, both of which contact the interior of a body passageway when the occlusion device is deployed. The anchor may also include a wire having two ends, neither of which contacts the interior of a body passageway when the occlusion device is deployed. The occluder may fit at least partially within the anchor after the occlusion device is deployed within a body passageway. The occluder may trail the anchor after the occlusion device is deployed within a body passageway. The filament may be polypropylene. The occluder may include a coil.

In another respect, the present invention is a device useful in blocking flow through a passageway in a living being. The device includes an anchor having a variable shape. The device also includes a coil attached to the anchor at a first location. The coil has a passageway. The device also includes a filament attached to the anchor at a second location. The filament extends away from the second location through at least a portion of the passageway to a location from which the filament can be manipulated. Manipulation of the filament alters the variable shape of the anchor.

In other respects, the anchor may include a wire having two ends, each of which is covered by a coil tip. The wire may be stainless steel. The wire may be a nickel titanium alloy. The anchor may include multiple acute bends. The anchor may include two ends, both of which contact the interior of a body passageway when the device is deployed. The coil may fit at least partially within the anchor after the device is deployed within a body passageway. The coil may trail the anchor after the occlusion device is deployed within a body passageway. The filament may interconnect multiple acute bends of the anchor.

In another respect, the present invention is a retrievable device useful in blocking flow through a vessel. The device includes a wire forming at least part of an anchor that has a variable shape. The device includes a coil attached to the anchor at a first location. The coil has a passageway that houses at least a portion of the wire. The device includes a filament attached to the anchor at a second location. The filament extends away from the second location through at least a portion of the passageway to a location from which the filament can be manipulated. Manipulation of the filament alters the variable shape of the anchor.

In another respect, the present invention is a device useful for occluding a passageway in a body. The device includes a wire having two ends. The wire forms a closed structure. The device also includes an occluder attached to the wire proximate at least one of the ends. The closed structure is elongated so as to facilitate delivery of the device into a body passageway, and, after deployment, the closed structure contacts the interior of the body passageway in order to prevent the device from migrating. The occluder may include a coil, and, after deployment, the closed structure may define a space occupied at least in part by the coil. The occluder may include a coil that trails the closed structure after the device is deployed within a body passageway. The closed structure may be characterized by a bend that facilitates loading the device into a catheter for delivery. The occluder may include a coil. The coil may include a coil passageway and the wire may extend at least partially into the coil passageway. The wire may be a nickel titanium alloy. The occluder may include a coil to which one or more threads of polyester are attached. The device may also include a second closed structure operably coupled to the closed structure. The occluder may include a coil. The coil may include a coil passageway and the wire may extend at least partially into the coil passageway. The wire may be a nickel titanium alloy. The occluder may include a coil to which one or more threads of polyester are attached. The occluder may include a coil that trails the closed structure and the second closed structure after the device is deployed within a body passageway. Both closed structures may be characterized by bends that facilitate loading the device into a catheter for delivery.

### BRIEF DESCRIPTION OF THE DRAWINGS

The following drawings form part of the present specification and are included to further demonstrate certain aspects of the present occlusion devices. The present occlusion devices may be better understood by reference to one or more of these drawings in combination with the description of illustrative embodiments presented herein.
**FIG. 1** is a front view of one of the present occlusion devices and a deployment system.
**FIG. 2** is a front view of the occlusion device depicted in **FIG. 1** loaded into a delivery catheter.
**FIG. 3** depicts the interaction between a catheter, a pusher wire, and a coil used an occluder.
**FIGS. 4a****-d** depict various stages of compression of an anchor having multiple bends.
**FIG. 5** is a front view of the anchor depicted in **FIGS. 4a****-d** and an occluder attached thereto partially-deployed in a body passageway.
**FIG. 6** is a front view of an embodiment of the occlusion device depicted in **FIG. 5** that has a wire acting as a core within the passageway of the coil.
**FIG. 7** is a front view of another embodiment of the occlusion device depicted in **FIG. 5** that has a fibrous braid surrounding a bare wire for use an the occluder.
**FIG. 8** is a front view of an occlusion device deployed in a body passageway.
**FIGS. 9a****-b** illustrate the variable nature of the shape of an anchor of an occlusion device.
**FIG. 10** is a front view of part of an occlusion device in which the anchor has symmetrical bends.
**FIG. 11a** is a slightly perspective view of a partially-deployed occlusion device.
**FIG. 11b** is an enlarged view of the small loop shown in Fig. 11a.

### DESCRIPTION OF ILLUSTRATIVE EMBODIMENTS

The design of the present occlusion devices facilitates the surgeon's task of deploying the devices at locations within body passageways (e.g., blood vessels) from which the devices are unlikely to migrate. The present devices include anchors that may be configured in a variety of shapes, each of which are suited to securely contacting the interior of the body passageway in which the device is deployed. Occluders, such as coils, are attached to the anchors, including attachment through integral formation, to facilitate the occlusion of a chosen site within a body passageway. To further the occlusion process, strands of material such as polyester may be attached to the disclosed occluders. As a result of their unique shapes, the present occlusion devices are well-suited to creating occlusions in body passageways that are not tapered and in which the potential for migration is consequently very high, such as arteriovenous (a-v) fistulas, a-v malformations, aneurysms and pseudoaneurysms, patent ductus arteriosus, systemic-to-pulmonary collateral vessels and shunts, and varicocele.

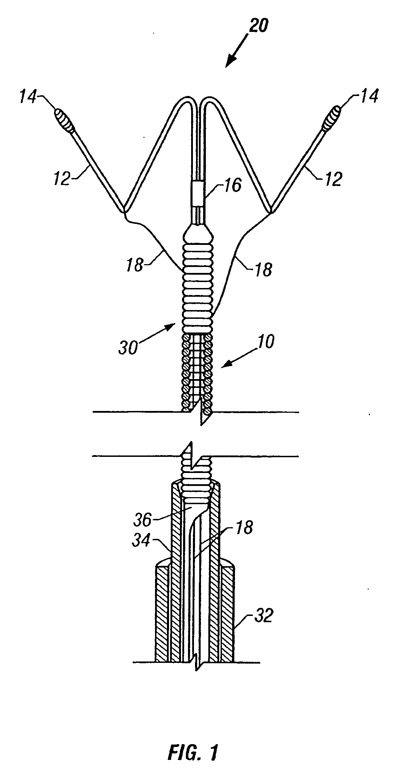

**FIG.** 1 depicts one embodiment of an occlusion device **10** that includes an anchor **20.** Anchor **20** includes two wires **12** that each have two acute bends. The exposed ends of the wires are covered with atraumatic tips **14.** Tips **14** may possess any shape suited to decreasing the potential that the covered wire ends will penetrate the wall of the passageway in which device **10** is deployed. For example, tips **14** may therefore be made from small pieces of coil. The materials from which tips **14** may be formed include those discussed below from which wires **12** may be formed.

Wires **12** are attached to each other at location 16 so as to facilitate the ability of anchor **20** to retain its variable shape after it is manipulated and eventually deployed. This attachment may be made using solder or welding, by crimping a third piece of material around the wires, or through the use of any other suitable means. Wires **12** are also shown attached to occluder **30,** which, as depicted, is a coil that has been substantially straightened in order to facilitate the deployment of occlusion device **10.** Such attachment may be made along any suitable length of wires **12** and any corresponding length of coil **30** using any of the foregoing types of attachment. Thus, as used herein, the term "first location" (or second location, etc.) in the phrase "at a first location," which phrase describes where the attachment of an anchor (e.g., anchor **20**) to an occluder (e.g., coil **30**) is made, means any length and not simply an immeasurably small point.

Filaments **18** are attached to both wires **12** of anchor **20** at one of the two acute bends in wires **12** as shown in **FIG. 1. FIG. 1** further shows that filaments **18** may then extend away from their attachment locations through gaps in coil **30** to the coil passageway, or lumen, within coil **30.** Filaments **18** may extend through at least a portion of this passageway to a location or locations (not shown) from which either or both may be manipulated. Filaments **18** may be made, for example, of polypropylene (e.g., PROLENE) or NYLON (available from Ethilon).

In another embodiment of occlusion device **10,** anchor **20** may be formed from a single wire having five acute bends, instead of two wires having two acute bends each. As a result, each time some aspect of wires **12** are discussed, it is to be understood that the discussion applies also to the foregoing single-wire embodiment of anchor **20.**

Wires **12** of anchor **20** depicted in **FIG. 1** may be formed of virtually any material compatible with the tissue of the body passageway in which the occlusion device will be placed. The material may be suitably rigid and elastic, such as stainless steel. The material may also be suitably rigid and elastic and capable of being programmed with either superelasticity or thermal shape memory. Accordingly, the material may, for example, be a nickel titanium alloy such as nitinoL In one embodiment, for example, nitinol possessing about 55 to 56 % Nickel, and 45 to 44 % Titanium, may be used for the wire or wires of anchor **20.** (Such nitinol wires are commercially available from Shape Memory Applications in Santa Clara, CA.) Such alloys can be heat treated to have either superelastic or thermal shape memory properties, depending on the type of heat treatment, as is well known by those of skill in the art. A wire that is superelastic may generally be bent into virtually any conceivable shape through the application of a force and will return to substantially, if not identically, its original shape when that force is removed. A wire that has thermal memory will generally be malleable below a certain temperature, but will take on a preprogrammed shape at some higher temperature. After taking on its preprogrammed shape, the wire having thermal memory will then generally act like a wire with superelasticity. Other alloys that may be used include FePt, FePd, and FeNiCoTi. These alloys may be heat treated to exhibit thermoelastic martensitic transformation, and, therefore, good thermal shape memory. Other alloys such as FeNiC, FeMnSi, and FeMnSiCrNi do not possess long-range order and undergo nonthermoelastic transformation, and, thus, may also be used. Additionally, some β-Ti alloys and iron-based alloys may also be used. These materials may be used for all of the present anchors.

Like wires **12,** coil **30** may be made from virtually any material compatible with the tissue of the body passageway in which the occlusion device will be placed. As a result, the materials described above that are suitable for forming wires **12** may also be used to form coil **30.** In addition, materials such as tantalum or platinum may also be used for coil **30.** Any suitable coil mentioned in the Background may be used in conjunction with anchor **20,** including the microcoils marketed by Medi-Tech or the Gianturco coils marketed by Cook, Inc. Coil **30** may be coated with thrombogenic material such as an electro-positive substance. Platinum is an example of an electro-positive substance that may be used either as the coil material or as a coil coating. Depending on the material used, coil **30** may be spring-biased or provided with superelasticity or thermal memory so as to assume any desired shape, including a helical shape, a rosette shape, or an irregular tangle, when unrestrained.

The size of wires **12** of anchor **20** will vary depending on the size of the body passageway into which the occlusion device will be deployed. Wires having diameters ranging from 0.003 inches ( 0,0076 cm) to 0.012 inches ( 0,0305 cm) in diameter may be used for most applications. In addition, when the occluder attached to the anchor is a coil, as in **FIG. 1**, a coil ranging in diameter from 0.03 to 0.05 inches (0.0762 to 0.127 cm) may be used for most applications.

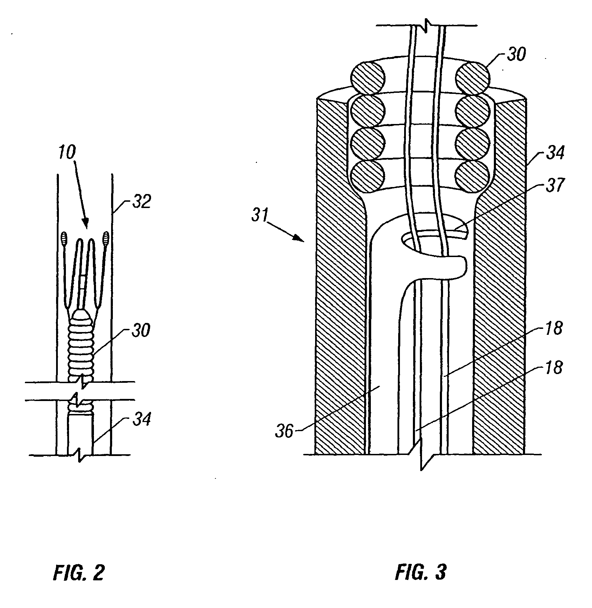

Turning to one manner of deploying the present occlusion devices, **FIG. 2** depicts occlusion device **10** loaded into delivery catheter **32.** A catheter is a medical term used to describe a hollow, flexible tube that may be inserted into a living being. Catheter **32** may be any adequate size. For some applications, a 5-French (F) outer diameter catheter having an internal diameter of 0.05 inches (0.127 cm) may be used for catheter **32.** Also depicted in **FIG. 2** is catheter **34,** which abuts the proximal end of coil **30.** "Proximal" refers to the end of the coil (or any other thing in question) that is closest (with respect to the other end of the coil) to the operator/surgeon. The term "distal" refers to the coil end that is the farthest from the operator. As depicted in both **FIGS. 1** and **3****,** catheter **34** may possess an internal diameter that decreases in size slightly as one moves away from the distal end of the catheter. As a result, the proximal end of the coil will not slide beyond that narrowed portion when an operator abuts the coil with the distal end of catheter **34.** Being able to stabilize coil **30,** or prevent it from moving, is useful because it may become desirable to retract, or pull back on, filaments **18,** as discussed below. During deployment, the operator/surgeon may lock catheter **34** to delivery catheter **32** together using locking mechanisms, such as Luer locks, which are well known in the art.

Within the lumen, or passageway, of catheter **34** is pusher wire **36** depicted in **FIGS. 1** and **3****.** Pusher wire **36** may be equipped with a cutting edge **37,** as depicted in **FIG. 3****,** in order to sever filaments **18** at the appropriate time. Pusher wire **36** and catheter **34** make up one embodiment of pusher system **31.** Pusher system **31** may include catheter **34,** catheter **34** and pusher wire **36,** or any device capable of preventing coil **30** from moving when resistance is applied to filaments **18** (i.e., when the operator/surgeon pulls back, or withdraws, filaments **18**). As with delivery catheter **32** and catheter **34,** pusher wire **36** may be locked to catheter **34** during deployment using any suitable locking mechanism. Similarly, filaments **18** may be locked to pusher wire **36** (or catheter **34** if only the same is used without pusher wire **36** as described above). By locking two devices together, one device cannot be moved relative to the other; they will move together.

Anchor **20,** like all of the present anchors, possesses a variable shape. This means that while anchor **20** possesses a relatively stable, unconstrained shape, that shape can be altered by applying some kind of force to the anchor in order to facilitate deployment of the occlusion device. For example, the acute bends in wires **12** of anchor **20** make it easy to reduce the size of anchor **20** by squeezing together, or compressing, wires **12.** Similarly, as described below, the proximal end of coil **30** may be held steady by pusher system **31** within delivery catheter **32** while filaments **18** are pulled back thereby forcing wires **12** to compress and reduce the size, or profile, of anchor **20.** In other words, manipulation of filaments **18** alters the variable shape of anchor **20.**

After occlusion device **10** is loaded into delivery catheter **32,** the operator/surgeon may utilize a guiding catheter (not shown) to facilitate guiding delivery catheter **32** to the target site within the body passageway. More specifically, if there is not enough room within delivery catheter **32** to insert a guidewire that can facilitate guiding the delivery catheter to the target location, a guiding catheter may be used for this purpose. That is, a guiding catheter may be introduced over a guidewire that has negotiated the body to the appropriate location within the body passageway, the guidewire may be removed, and the delivery catheter may be inserted into the guiding catheter and advanced to the target location. The guiding catheter may be removed after the delivery catheter is in place if the operator/surgeon so chooses. After delivery catheter **32** is satisfactorily positioned, occlusion device **10** is deployed. The distal portions of anchor **20** may be released by pushing, or advancing, the occlusion device with pusher system **31.** As a result, anchor **20** will contact the interior of the body passageway at multiple sites, thereby stabilizing the occlusion device and reducing the chance of migration. Pusher system **31** may then be held steady while delivery catheter **32** is gently pulled back, or withdrawn, thereby exposing the proximal portions of anchor **20.** Using this technique, anchor **20** can be precisely deployed.

Coil **30** may be deployed, and thus occlusion device **10** may be deployed, by using a combination of pushing the coil using pusher system **31** and retracting delivery catheter **32,** as well as by rotating delivery catheter **32.** Using these or similar maneuvers, the operator/surgeon may achieve the most compact arrangement of the occlusion device possible.

If deployment of occlusion device **10** is satisfactory, any locking mechanism used to couple catheter **34** to pusher wire **36,** if both are used, may be released. Pusher wire **36** may then be rotated such that cutting edge **37** severs any suitable length off of filaments **18.** Any suitable manner of severing filaments **18** may be used in the alternative to cutting edge **37** of pusher wire **36.** Pusher wire **36** may then be advanced, or moved distally, to push the proximal end of coil **30** from the lumen of catheter **34** to free the occlusion device from the deployment system.

If deployment of occlusion device **10** is not satisfactory, the operator/surgeon should maintain control of filaments **18,** and any portion of the device may be withdrawn into delivery catheter **32,** and the process started again. The operator/surgeon may position pusher system **31** against, or close to, the proximal end of coil **30** as delivery catheter **32** is advanced over the malpositioned portion of the occlusion device. If anchor **20** is misplaced, once coil **30** has been pulled back into delivery catheter **32,** any mechanism that is locking filaments **18** to any other aspect of the deployment system may be released. Then, while pusher system **31** is held steady, filaments **18** are pulled back to cause anchor **20** to contract such that it can be re-housed by delivery catheter **32.** Filaments **18** may then be re-locked to the appropriate device. Filaments **18** and pusher system **31** may then be held steady while delivery catheter **32** is advanced (i.e., pushed or moved forward) to enclose anchor **20.** The deployment process may then be repeated as necessary to achieve a satisfactory deployment of occlusion device **10.**

The present occlusion devices are advantageous because any displacement of the present occlusion device (including of the anchor) can be corrected during any phase of deployment. As a result, deployment becomes completely controlled. Further, the relationship between the coil and the pusher system makes it possible to achieve the most compact arrangement of the coil possible, thereby furthering the effectiveness of the occluder and of the occlusion device generally.

Throughout this disclosure, like reference numerals indicate like features.

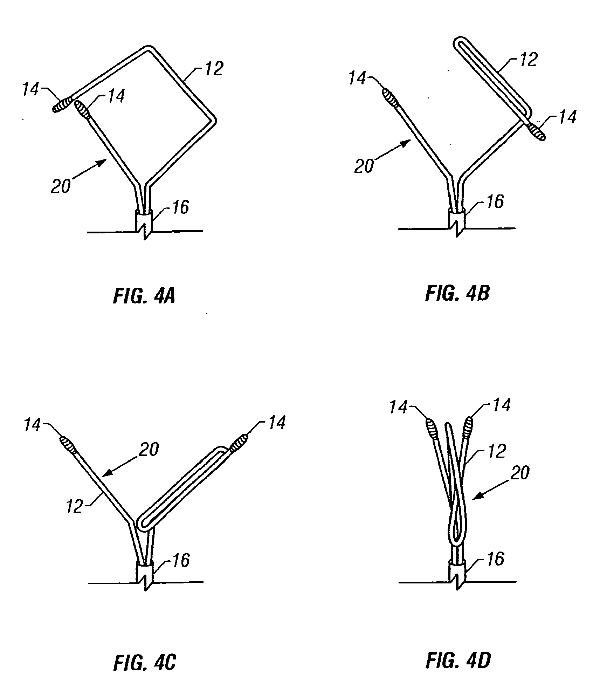

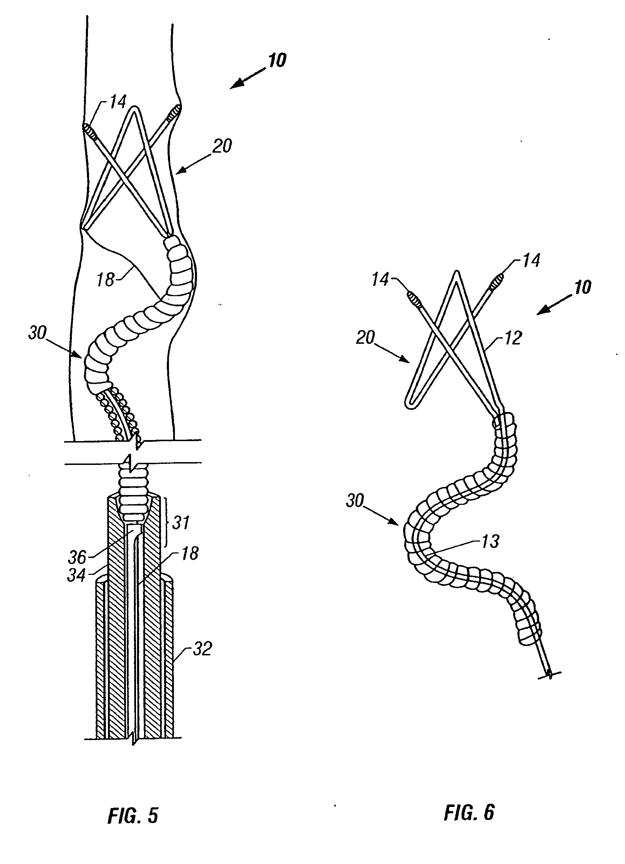

The anchors of the present occlusion devices may possess a variety of shapes. **FIGS. 4a**-**d** illustrate the various states of deployed anchor **20** (illustrated in **FIG. 5**) as anchor **20** is being folded from its unconstrained state (**FIG. 4a**) to its constrained state (**FIG. 4d**). In this embodiment of anchor **20,** it is worth noting that the unconstraint shape of anchor **20** (**FIG. 4a**) differs from the deployed shape of anchor **20** depicted in **FIG. 5**. Anchor **20** depicted in FIGS. **4a-5** may include one or two wires **12** like anchor **20** depicted in **FIG. 1**. These two wires, or the segments of one wire bent at a very acute angle, may be attached to each other at location **16** as described above. **FIG. 5** illustrates occlusion device **10** having only one filament **18** as it is being deployed within a body passageway. As depicted, pusher system **31** along with deliver catheter **32** may be used to facilitate deployment.

In any embodiment of the present occlusion devices, including those depicted in **FIGS. 1** and **5****,** a wire **12** of anchor **20** may extend partially or completely along the length of the passageway, or lumen, within coil **30,** sometimes referred to herein as the coil passageway. In such an embodiment, anchor **20** will usually consist of two wires **12,** one of which may be attached to coil **30** near the same portion of coil **30** to which the shorter wire of anchor **20** is attached. Alternatively, the longer wire extending into the coil passageway may be attached at any suitable location along the coil passageway, including at multiple, spaced-apart locations. Such an embodiment of an occlusion device **10** is depicted in **FIG. 6**. The portion of the wire extending into the coil passageway may be referred to as occluder core **13.** When wire **12,** and hence occluder core **13,** is made from a nickel titanium alloy such a nitinol, core **13** may be preprogrammed to take on any given shape. The preprogramming may impart either superelastic or thermal memory properties to core **13,** which properties are discussed above. This feature allows coil **30** to take on shapes that can better fill a target location within a body passageway so as to result in quicker occlusion. In addition, any portion of the occluder (e.g., coil) passageway may be occupied by core **13.** Leaving a portion of the coil empty near its proximal end may facilitate the operator/surgeon's task of compactly arranging coil **30** within the space defined by deployed anchor **20.** Core **13** may be tapered along any portion of its length to facilitate this task as well. In other words, core **13** may be continuously tapered from the proximal end of anchor **20** (where it meets anchor **20**) to its own proximal end, or it may be both uniform in width from the proximal end of anchor **20** to a location proximal thereof and tapered from the latter location to its own proximal end.

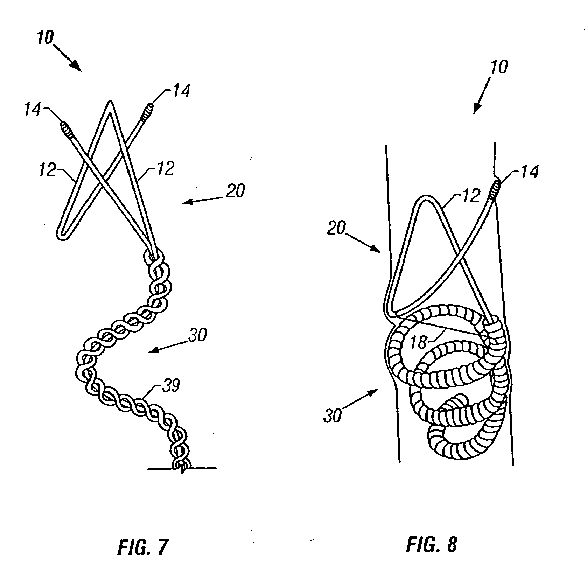

Another embodiment of occlusion device **10** is depicted in **FIG. 7****.** In this embodiment, anchor **20** may include the same arrangement of wires **12** that is depicted in **FIG. 6****.** With this embodiment, as with the embodiment of occlusion device **10** depicted in **FIG. 6**, wires **12** may be connected to each other for stability at or near the proximal end of the shorter wire. The difference between the embodiment of occlusion device **10** and of the occlusion device shown in **FIG. 6** is that a coil is not used for occluder **30.** Instead, and as shown in **FIG. 7**, the longer of the two wires may be covered, partially or completely, with a fibrous material, such as braided polyester, braided DACRON, braided silk, or the like. Fibrous material **39** may be secured to the longer of the two wires **12** using any suitable means, such as glue, resin, etc.

Another embodiment of occlusion device **10** is depicted in **FIG. 8**. As shown, anchor **20** of occlusion device **10** includes a single wire **12** having two acute bends. Prior to loading this embodiment of occlusion device **10** into a delivery catheter, the unconstrained shape of anchor **20** similar to that of a triangle, where atraumatic tip **14** is located near the distal end of coil **30**.

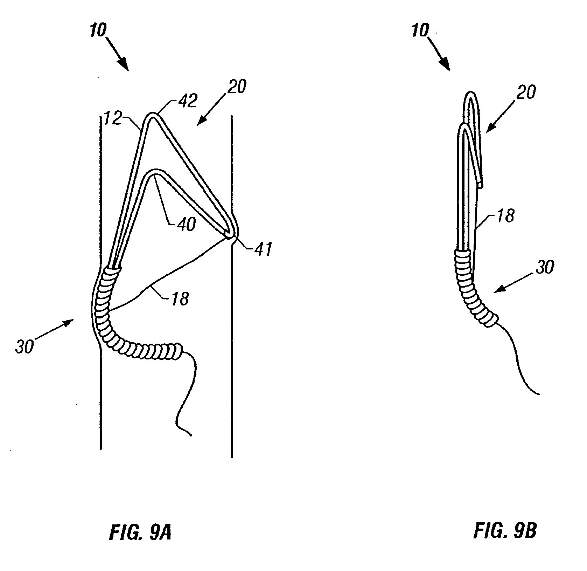

Additional embodiments of anchor **20** appear in **FIGS. 9a-10**. These embodiments of anchor 20 include one wire that is bent at least three times at acute angles and does not include any exposed wire ends. It is to be understood that the lengths of coil **30** depicted in FIGS. **9a-10** are truncated (i.e., shorter than normal) for ease of illustration, as is the case throughout the present figures. As illustrated in **FIG. 9a**, anchor **20** includes wire **12,** which is bent at locations **40, 41,** and **42.** As shown, bend **41** has the appearance of a beak. The ends of wire **12** (not shown) may be secured to each other and attached to coil **30** as described above. **FIG. 9b** illustrates occlusion device **10** depicted in **FIG. 9a** in its constrained form, ready for insertion into a delivery catheter.

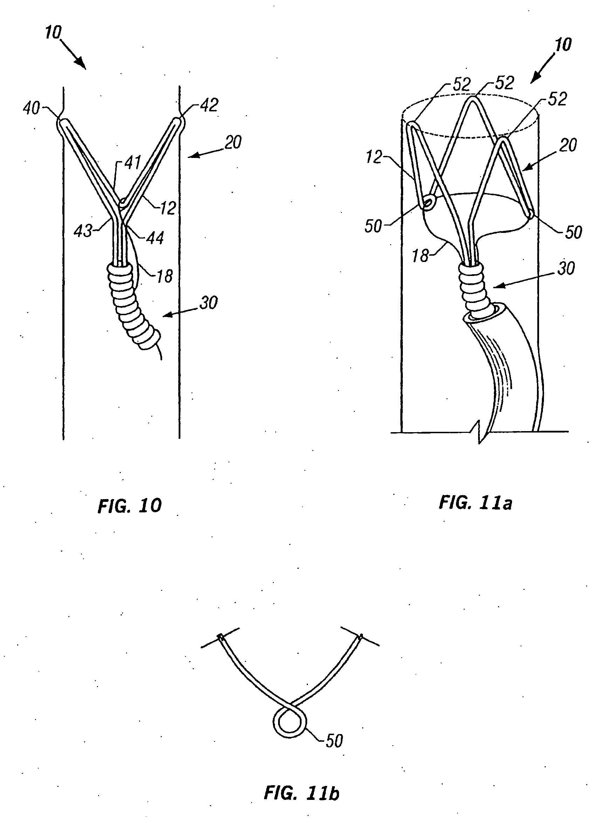

In additions to bends **40, 41,** and **42,** wire **12** forming anchor **20** may be bent at any other suitable angles to better ensure that anchor **20** will serve to prevent the migration of occlusion device **10.** For example, as shown in **FIG. 10****,** wire **12** may be bent at locations **43** and **44** to cause anchor to contact the interior of a body passageway in additional locations. Additionally, the lengths of the segments of wire between the bends in wire **12** may also be varied as desired. As shown in **FIG. 10****,** for example, the various lengths of wire between bends **41** and **42,** in between bends **41** and **40,** are virtually identical, as are the lengths of wire between, for example, bends **40** and **43** and between bends **42** and **44.** As a result, anchor **20** is symmetrically shaped.

In yet another embodiment of the anchor that may be used for present occlusion devices, **FIG. 11a** depicts a zigzag-shaped anchor **20** that includes a single wire **12** having multiple bends and small loops **50** (see **FIG. 11b** for an enlarged view of small loop **50**). Like the other single wire embodiments of the present anchors, the ends of wire **12** may be secured to each other and to coil **30** at or near the distal end of coil **30.** While three bends **52** characterize the distal portion of anchor **20** in **FIG. 11a****,** it is to be understood that fewer or greater than three bends **52** may be used. However, three bends **52** are well-suited to evenly distributing the expansile force of anchor **20** against the interior of body passageway in which it is deployed. Specifically, the expansile force of this configuration may be evenly distributed along the circumference of the effected body passageway. As shown, filament **18** may be threaded through small loops **50** and into the coil passageway of coil **30.** It should be noted that as with all of the present occlusion devices, filament(s) **18** may enter the coil passageway beginning at the distal end of coil **30** or at any point along coil **30** through an appropriately situated gap. It is also to be understood that, although it is not shown, upon deployment, coil **30** may fill all or at least a part of the space defined by anchor **20.** Alternatively, coil **30** may trail (i.e., be located proximately of) anchor **20** after occlusion device **10** is deployed.

As discussed generally above, the thrombogenicity of the present occlusion devices may be improved by providing the occluders (e.g., coils) with strands of material such as polyester. The strands may be placed in bundles at every 1.5 to 2.0 centimeters along the occluder, for the entire length of the occluder. The strands may also be attached to the relevant occluder, particularly with respect to coils, without forming bundles and without leaving empty sections, or spaces, along the occlude. In another embodiment, fibrous material may be braided around a particular occluder, especially coils, forming an even layer on its surface.

All of the present occlusion devices disclosed and claimed herein can be made and executed without undue experimentation in light of the present disclosure. While the techniques of this invention have been described in terms of specific embodiments, it will be apparent to those of skill in the art that variations may be applied to the present occlusion devices without departing from the scope of the invention.

### REFERENCES

1. Gianturco C, Anderson JH, Wallace S. Mechanical device for arterial occlusion. AJR 1975; 124.-428-435.
2. Chuang VP, Wallace S, Gianturco, C. A new improved coil for tapered-tip catheter for arterial occlusion. Radiology 1980; 135:507-509.
3. Hawkins J, Quisling RG, Mickle JP, Hawkins IF. Retrievable Gianturco-coil introducer. Radiology 1986; 158:262-264.
4. Remy-Jardin M, Wattinne L, Remy J. Transcatheter occlusion of pulmonary arterial circulation and collateral supply: failures, incidents, and complications. Radiology 1991; 180:699-705.
5. White R, Lynch-Nyhan A, Terry P et al. Pulmonary arteriovenous malformations: techniques and long-term outcome of embolotherapy. Radiology 1988; 169:663:669.
6. Keller FS, Rösch J, Barker AF, Nath PH. Pulmonary arteriovenous fistulas occluded by percutaneous introduction of coil springs. Radiology 1984; 152:373:375.
7. Butto F, Hunter DW, Castaneda-Zuniga W, Amplatz K. Coil-in-coil technique for vascular embolization. Radiology 1986,161:554-555.
8. Castenada-Zuniga WR, Tadavarthy SM, Gonzalez R, Rysavy J, Amplatz K. Single barbed stainless steel coils for venous occlusion. A simple but useful modification. Invest Radiol 1982; 17:186-188.
9. Lund G, Cragg AH, Rysavy JA, Castaneda F, Castenada-Zuniga WR, Amplatz K Detachable stainless-steel spider. Radiology 1983; 148;567-568.
16. Castenada-Zuniga WR, Tadavarthy SM, Galliani CA, Laerum F, Schwarten DE, Amplatz K. Experimental venous occlusion with stainless-steel spiders. Radiology 1981; 141:238-242.
11. Ing FF, Bierman PZ. Percutaneous transcatheter coil occlusion of the patent ductus arteriosus aided by the nitinol snare: further observations. Cardiovasc Intervent Radiol 1995; 18:222-226.
12. Sommer RJ, Gutierrez A,. Lai WW, Pamess. Use of preformed nitinol snare to improve transcatheter coil delivery in occlusion of patent ductus arteriosus. Am j Cardiol; 1994, 74:836-839.
13. Zubillaga AF, Guglielimi G, Vinuela F, Duckwiler GR. Endovaesulur occlusion of intracranial aneurysams with electrically detachable coils: correlation of aneurysm neck size and treatment results. AJNR 1994; 15:815-820.
14. Guglielimi G, Vinuela F, Duckwiler G, Dion J, Stocker A. High-flow, small-hole arteriovenous fistulas treatment with electrodetachable coils. AJNR 1995; 16:325-328.
15. Vinuela F, Duckwiler G, Mewed IvL Guglielad detachable coil embolirAtion of acute intracramal aneurysm penoperative anatomical and clinical outcome in 403 patients. J Neurosurg 1997,86.475482
16. Marks MP, Chee H, Liddel RP, Steinberg GK, Panahian N, Lane B. A mechanically detachable coil for the treatment of aneurysms and occlusion of blood vessel. AJNR 1994;15:821-827.
17. Reidy JF, Qureshi SA. Interlocking detachable platinum coils, a controlled embolization device: early clinical experience. Cardiovasc intervent Radiol 1996; 19:85-90.
18. Uzun O, Hancock S, Parsons JM, Dickinson DF, Gibbs JL. Transcatheter occlusion of the arterial duct with Cook detachable coils: early experience. Heart 1996; 76:269-273.
19. Tometzki AJ, Arnold R, Peart I et al. Transcatheber occlusion of the patent ductus arteriosus with Cook detachable coils. Heart 1996, 76:531-535.
20. Dutton JAE, Jackson JE, Hughes JMB et al. Pulmonary arteriovenous malformations Results of treatment with coil embolization in 53 patients. AJR 1995; 165:1119-1125.
21. Hughes SR, Graves VB, Kesava PP, Rappe AH. The effect of flow arrest on distal embolic events during arterial occlusion with detachable coils: a canine study. AJNR 1996;17:685-691.
22. Chuang VP, Szwarc I. The coil baffle in the experimental occlusion of large vascular structures. Radiology 1982;143:25-28.
23. Levey DS, Teitelbaum GP, Finck EJ, Pentecost MJ. Safety and efficacy of transcatheter embolization of axfllary and shoulder arterial injuries. JVIR 1991; 2:99-104.
24. Celiker A. Qureshi SA, Bilgic A et al. Transcatheter closure of patent arterial ducts using controlled-relessed coils. Eur Heart J 1997;18:450-454.
25. Mauro MS. Jaques P. Transcatheter management ofpseudoareurysms complicating pancreatitis. JVIR 1991; 2:527-532.
26. Yedlicka JW, Bjarnawn K Hunter DW, Castenada Zuniga WR, AmPlRtz K. Persistent pneurnothorax following embolization of a Pulmonary arteriovenOus malformation. JVIR 1994;5:887-889.
27. Krichenko A, Benson LN, Burows P, Moes CAF, McLaughlin P, Freedom RM. Angiographic classification of the isolated, persistently patent ductus arteriosus and impUcations for percutaneous catheter occlusion. Am J Cardiol 1989; 63:877-880.
28. Cambier PA, Kirby WC, Wortham DC, Moore JW. Percutaneous closure of the small (<2,5 mm) patent ductus arteriosus using coil embolization. Am J Cardiol 1992; 69:815-816.
29. Lloyd TF, Fedderly R, Mendelsohn AM, Sandhu SY, Beekman RH. Transcatheter occlusion of patent ductus arteriosus with Gianturco coils. Circulation 1993; 88:1412-1420.
30. Hijazi ZM, Geggel RL. Trarnscatheter closure of large patent ductus arterimus (> or = 4 mm) with multiple Gianturco coilv Immediate and mid- results. Heart 1996; 76:536-540.
31. Dalvi B, Goyal V, Narula D, Kulkarni H, Ramakantan R. New technique using temporary balloon occlusion for transcatheter closure of patent ductus arteriosus with Gianturco coils. Cathet Cardiovasc Diagn 1997; 41:62-70.
32. Galalo de Moor M, Fadley F at al. Problems encountered during introduction of Gianturco coils for transcatheter occlusion of the patent arterial duct. Eur Heart J. 1997, 18:625-630.
33. Lois JF, Gomes AS, Smith DC, Laks H. Systemic-to-pulmonary collateral vessels and shunts: treatment with embolization. Radiology 1988;169:671-676.
34. Perry SB, Rome J, Keane JF at al. Transcatheter. Closure of coronary artery fistulas. Am Coil Cardiol 1992; 20:205-209.
35. Chomyn JJ, Craven WM Groves BM, Durham JD. Percutaneous removal of a Gianturco coil from the pulmonary artery with use of flexible intravascular forceps. JIR 1991; 2:105-106.
36. Smith DC, Kohne RE, Taylor PC. Steel coil embolization supplementing filter placement in a patient with a duplicated inferior vena cave. JVIR 1992; 3:577-580.
37. Kónya A, Wright KC, Wallace S. Anchoring coil embolization in a high-flow arterial model: A pilot study. JVIR 1998; 9:249-254.
38. Maeda M, Okada A, Takamura M, Kawata S, Tomoda K, Tomiyama N, Curz MA, Johhoh T, Murakami T, Hamada S, Oi K Nakamura H. "Coil Anchor": A new device to improve coil embolization: Experimental and Initial Clinical Use. 7th International Symposium on Interventional Radiology & New Vascular Imaging and 28th Annual Meeting of the Japanese Society of Angiography & Interventional Radiology. Osaka, Japan, May 16-19,1999. Abstract No. 20 in Cardiovascular and Interventional Radiology 1999; 22(Suppl):S54.

## Claims

1. An occlusion device **(10)** to facilitate the occlusion of a chosen site within a body passageway, the occlusion device **(10)** comprising:
an anchor **(20)** having a variable shape;
an occluder **(30)** attached to the anchor **(20)** at a first location; and
a filament **(18)** attached to the anchor **(20)** at a second location;
wherein manipulation of the filament **(18)** alters the variable shape of the anchor **(20).**

2. The occlusion device **(10)** of claim 1, wherein the anchor includes a wire **(12).**

3. The occlusion device **(10)** of claim 2, wherein the wire **(12)** is stainless steel.

4. The occlusion device **(10)** of claim 2, wherein the wire **(12)** is a nickel titanium alloy.

5. The occlusion device **(10)** of claim 1, wherein the anchor **(20)** includes a wire **(12)** having multiple acute bends.

6. The occlusion device **(10)** of claim 1, wherein the anchor **(20)** includes a wire **(12)** having two ends, both of which contact the interior of a body passageway when the occlusion device **(10)** is deployed.

7. The occlusion device **(10)** of claim 1, wherein the anchor includes a wire **(12)** having two ends, neither of which contacts the interior of a body passageway when the occlusion device **(10)** is deployed.

8. The occlusion device **(10)** of claim 1, wherein the occluder fits at least partially within the anchor **(20)** after the occlusion device **(10)** is deployed within a body passageway.

9. The occlusion device **(10)** of claim 1, wherein the occluder **(30)** trails the anchor **(20)** after the occlusion device **(10)** is deployed within a body passageway.

10. The occlusion device **(10)** of claim 1, wherein the filament **(18)** is polypropylene.

11. The occlusion device **(10)** of claim 1, wherein the occluder **(30)** includes a coil **(30).**

12. The occlusion device **(10)** of claim 1, wherein the coil **(30)** has a passageway, and the filament **(18)** extends away from the second location through at least a portion of the passageway to a location from which the filament **(18)** can be manipulated.

13. The occlusion device of claim 12, wherein the anchor **(20)** includes a wire **(12)** having two ends, each of which is covered by a coil tip **(14).**

14. The occlusion device **(10)** of claim 13, wherein the wire **(12)** is stainless steel.

15. The occlusion device **(10)** of claim 13, wherein the wire **(12)** is a nickel titanium alloy.

16. The occlusion device **(10)** of claim 12, wherein the anchor **(20)** includes multiple acute bends.

17. The occlusion device **(10)** of claim 12, wherein the anchor **(20)** includes two ends, both of which contact the interior of a body passageway when the device **(10)** is deployed.

18. The occlusion device **(10)** of claim 12, wherein the coil **(30)** fits at least partially within the anchor **(20)** after the device is deployed within a body passageway.

19. The occlusion device **(10)** of claim 12, wherein the coil **(30)** trails the anchor **(20)** after the occlusion device **(10)** is deployed within a body passageway.

20. The occlusion device **(10)** of claim 12, wherein the filament **(18)** interconnects multiple acute bends of the anchor **(20).**

21. The occlusion device **(10)** of claim 1, wherein the occlusion device **(10)** is retrievable and further comprises
a wire **(12)** forming at least part of the anchor **(20)**; and
a coil **(30)** attached to the anchor **(20)** at a first location, the coil **(30)** having a passageway that houses at least a portion of the wire **(12)**;
wherein the filament **(18)** extends away from the second location through at least a portion of the passageway to a location from which the filament **(18)** can be manipulated.

## Patentansprüche

1. Eine Okklusionsvorrichtung **(10),** um die Okklusion einer ausgewählten Stelle innerhalb eines Körperdurchgangs zu ermöglichen, wobei die Okklusionsvorrichtung **(10)** das Folgende umfasst:
einen Anker **(20),** der eine variable Form hat;
einen Verschluss (Occluder) **(30),** der am Anker **(20)** an einer ersten Stelle angebracht ist; und
einen Faden (Filament) **(18),** der am Anker **(20)** an einer zweiten Stelle angebracht ist;
wobei die Manipulation des Fadens **(18)** die variable Form des Ankers **(20)** verändert.

2. Die Okklusionsvorrichtung **(10)** nach Anspruch 1, wobei der Anker einen Draht **(12)** einschließt.

3. Die Okklusionsvorrichtung **(10)** nach Anspruch 2, wobei der Draht **(12)** rostfreier Stahl ist.

4. Die Okklusionsvorrichtung **(10)** nach Anspruch 2, wobei der Draht **(12)** eine Nickel-Titan-Legierung ist.

5. Die Okklusionsvorrichtung **(10)** nach Anspruch 1, wobei der Anker **(20)** einen Draht **(12)** einschließt, der mehrere scharfe Krümmungen hat.

6. Die Okklusionsvorrichtung **(10)** nach Anspruch 1, wobei der Anker **(20)** einen Draht **(12)** einschließt, der zwei Enden hat, von denen beide das Innere eines Körperdurchgangs berühren, wenn die Okklusionsvorrichtung **(10)** eingesetzt wird.

7. Die Okklusionsvorrichtung **(10)** nach Anspruch 1, wobei der Anker **(20)** einen Draht **(12)** einschießt, der zwei Enden hat, von denen keines das Innere eines Körperdurchgangs berührt, wenn die Okklusionsvorrichtung **(10)** eingesetzt wird.

8. Die Okklusionsvorrichtung **(10)** nach Anspruch 1, wobei der Verschluss mindestens teilweise innerhalb des Ankers **(20)** passt, nachdem die Okklusionsvorrichtung **(10)** innerhalb eines Körperdurchgangs eingesetzt wurde.

9. Die Okklusionsvorrichtung **(10)** nach Anspruch 1, wobei der Verschluss **(30)** den Anker **(20)** nachzieht, nachdem die Okklusionsvorrichtung **(10)** innerhalb eines Körperdurchgangs eingesetzt wurde.

10. Die Okklusionsvorrichtung **(10)** nach Anspruch 1, wobei der Faden **(18)** Polypropylen ist.

11. Die Okklusionsvorrichtung **(10)** nach Anspruch 1, wobei der Verschluss **(30)** eine Spule **(30)** einschließt.

12. Die Okklusionsvorrichtung **(10)** nach Anspruch 1, wobei die Spule **(30)** einen Durchgang hat, und der Faden **(18)** sich aus der zweiten Stelle heraus durch mindestens einen Bereich des Durchgangs zu einer Stelle erstreckt, von der der Faden **(18)** manipuliert werden kann.

13. Die Okklusionsvorrichtung **(10)** nach Anspruch 12, wobei der Anker **(20)** einen Draht **(12)** einschließt, der zwei Enden hat, von denen jedes durch eine Spulenspitze **(14)** bedeckt ist.

14. Die Okklusionsvorrichtung **(10)** nach Anspruch 13, wobei der Draht **(12)** rostfreier Stahl ist.

15. Die Okklusionsvorrichtung **(10)** nach Anspruch 13, wobei der Draht **(12)** eine Nickel-Titan-Legierung ist.

16. Die Okklusionsvorrichtung **(10)** nach Anspruch 12, wobei der Anker **(20)** mehrere scharfe Krümmungen einschließt.

17. Die Okklusionsvorrichtung **(10)** nach Anspruch 12, wobei der Anker **(20)** zwei Enden einschließt, von denen beide das Innere eines Körperdurchgangs berühren, wenn die Vorrichtung **(10)** eingesetzt wird.

18. Die Okklusionsvorrichtung **(10)** nach Anspruch 12, wobei die Spule **(30)** mindestens teilweise innerhalb des Ankers **(20)** passt, nachdem die Vorrichtung innerhalb eines Körperdurchgangs eingesetzt wurde.

19. Die Okklusionsvorrichtung **(10)** nach Anspruch 12, wobei die Spule **(30)** den Anker **(20)** nachzieht, nachdem die Okklusionsvorrichtung **(10)** innerhalb eines Körperdurchgangs eingesetzt wurde.

20. Die Okklusionsvorrichtung **(10)** nach Anspruch 12, wobei der Faden **(18)** mehrere scharfe Krümmungen des Ankers **(20)** untereinander verbindet.

21. Die Okklusionsvorrichtung **(10)** nach Anspruch 1, wobei die Okklusionsvorrichtung **(10)** rückholbar ist und ferner das Folgende umfasst:
einen Draht **(12),** der mindestens einen Teil des Ankers **(20)** bildet; und
eine Spule **(30),** die am Anker **(20)** an einer ersten Stelle angebracht ist, wobei die Spule **(30)** einen Durchgang hat, der mindestens einen Bereich des Drahtes **(12)** beherbergt;
wobei sich der Faden **(18)** aus der zweiten Stelle heraus durch mindestens einen Bereich des Durchgangs zu einer Stelle erstreckt, von der der Faden **(18)** manipuliert werden kann.

## Revendications

1. Dispositif d'occlusion (10) pour faciliter l'occlusion d'un site choisi à l'intérieur d'une voie de passage corporelle, le dispositif d'occlusion (10) comprenant :
un ancrage (20) ayant une forme variable ;
un obturateur (30) fixé à l'ancrage (20) à un premier emplacement ; et
un filament (18) fixé à l'ancrage (20) à un second emplacement ;
dans lequel la manipulation du filament (18) modifie la forme variable de l'ancrage (20).

2. Dispositif d'occlusion (10) selon la revendication 1, dans lequel l'ancrage comprend un fil (12).

3. Dispositif d'occlusion (10) selon la revendication 2, dans lequel le fil (12) est en acier inoxydable.

4. Dispositif d'occlusion (10) selon la revendication 2, dans lequel le fil (12) est un alliage de nickel-titane.

5. Dispositif d'occlusion (10) selon la revendication 1, dans lequel l'ancrage (20) comprend un fil (12) ayant plusieurs plis aigus.

6. Dispositif d'occlusion (10) selon la revendication 1, dans lequel l'ancrage (20) comprend un fil (12) ayant deux extrémités, dont les deux sont en contact avec l'intérieur d'une voie de passage corporelle lorsque le dispositif d'occlusion (10) est déployé.

7. Dispositif d'occlusion (10) selon la revendication 1, dans lequel l'ancrage comprend un fil (12) ayant deux extrémités, dont aucune n'est en contact avec l'intérieur d'une voie de passage corporelle lorsque le dispositif d'occlusion (10) est déployé.

8. Dispositif d'occlusion (10) selon la revendication 1, dans lequel l'obturateur s'adapte au moins partiellement à l'intérieur de l'ancrage (20) après que le dispositif d'occlusion (10) a été déployé à l'intérieur d'une voie de passage corporelle.

9. Dispositif d'occlusion (10) selon la revendication 1, dans lequel l'obturateur (30) suit l'ancrage (20) après que le dispositif d'occlusion (10) a été déployé à l'intérieur d'une voie de passage corporelle.

10. Dispositif d'occlusion (10) selon la revendication 1, dans lequel le filament (18) est en polypropylène.

11. Dispositif d'occlusion (10) selon la revendication 1, dans lequel l'obturateur (30) comprend une spire (30).

12. Dispositif d'occlusion (10) selon la revendication 1, dans lequel la spire (30) a une voie de passage, et le filament (18) s'étend à distance du second emplacement en passant au moins par une partie de la voie de passage jusqu'à un emplacement à partir duquel le filament (18) peut être manipulé.

13. Dispositif d'occlusion selon la revendication 12, dans lequel l'ancrage (20) comprend un fil (12) ayant deux extrémités, dont chacune est recouverte par une pointe (14) de spire.

14. Dispositif d'occlusion (10) selon la revendication 13, dans lequel le fil (12) est en acier inoxydable.

15. Dispositif d'occlusion (10) selon la revendication 13, dans lequel le fil (12) est un alliage de nickel-titane.

16. Dispositif d'occlusion (10) selon la revendication 12, dans lequel l' ancrage (20) comprend plusieurs plis aigus.

17. Dispositif d'occlusion (10) selon la revendication 12, dans lequel l' ancrage (20) comprend deux extrémités, dont les deux sont en contact avec l'intérieur d'une voie de passage corporelle lorsque le dispositif (10) est déployé.

18. Dispositif d'occlusion (10) selon la revendication 12, dans lequel la spire (30) s'adapte au moins partiellement à l'intérieur de l'ancrage (20) après que le dispositif a été déployé à l'intérieur d'une voie de passage corporelle.

19. Dispositif d'occlusion (10) selon la revendication 12, dans lequel la spire (30) suit l'ancrage (20) après que le dispositif d'occlusion (10) a été déployé à l'intérieur d'une voie de passage corporelle.

20. Dispositif d'occlusion (10) selon la revendication 12, dans lequel le filament (18) interconnecte plusieurs plis aigus de l'ancrage (20).

21. Dispositif d'occlusion (10) selon la revendication 1, dans lequel le dispositif d'occlusion (10) est récupérable et comprend en outre :
un fil (12) formant au moins une partie de l'ancrage (20) ; et
une spire (30) fixée à l'ancrage (20) à un premier emplacement, la spire (30) ayant une voie de passage qui loge au moins une partie du fil (12) ;
dans lequel le filament (18) s'étend à distance du second emplacement en passant au moins par une partie de la voie de passage jusqu'à un emplacement à partir duquel le filament (18) peut être manipulé.
